# EUROPÄISCHE PATENTSCHRIFT

(11) **EP 2 304 087 B1**
(45) Veröffentlichungstag und Bekanntmachung des Hinweises auf die Patenterteilung: **24.04.2013**
(21) Anmeldenummer: 09771885.2
(22) Anmeldetag: 20.05.2009
(51) Int. Cl.: D01H 13/22, D01H 13/26, B65H 63/06

(54) **VORRICHTUNG ZUR ERFASSUNG VON PARAMETERN AN EINEM FADENFÖRMIGEN PRÜFGUT**
DEVICE FOR DETECTING PARAMETERS ON A THREAD-SHAPED TEST SPECIMEN
DISPOSITIF DE SAISIE DE PARAMÈTRES SUR UN PRODUIT FILIFORME À L'ESSAI

(30) Priorität: 02.07.2008 CH 10252008
(43) Veröffentlichungstag der Anmeldung: 06.04.2011
(73) Patentinhaber: Uster Technologies AG, 8610 Uster (CH)
(72) Erfinder: JOSS, Rolf, CH-8810 Horgen (CH)
(86) Internationale Anmeldenummer: PCT/CH2009/000168
(87) Internationale Veröffentlichungsnummer: WO 2010/000078

(56) Entgegenhaltungen:
- EP-A- 0 761 853
- EP-A1- 1 359 108
- EP-A1- 1 808 690
- EP-B- 0 945 533
- WO-A-00/07921
- DE-A1- 3 929 895
- US-A- 3 009 101

## Beschreibung

### FACHGEBIET

Die vorliegende Erfindung liegt auf dem Gebiet der von Prüfung von fadenförmigen, vorzugsweise textilen Gebilden wie Garn. Sie betrifft eine Vorrichtung zur Erfassung von Parametern an einem fadenförmigen Prüfgut, gemäss dem Oberbegriff des ersten Patentanspruchs. Ihr bevorzugtes Einsatzgebiet ist die Garnreinigung auf Spinn- oder Spulmaschinen.

### STAND DER TECHNIK

Es ist eine Vielzahl verschiedenartiger elektronischer Vorrichtungen zur Prüfung von Garn bekannt. Sie lassen sich nach ihrer Anwendung in die beiden Klassen Laborprüfung (offline) und Prüfung während des Produktionsprozesses (online) einteilen. Für die Anwendung der vorliegenden Erfindung sind, ohne Einschränkung der Allgemeinheit, die letzteren besonders interessant. Unter den Online-Prüfgeräten wiederum interessieren hier besonders die so genannten Garnreiniger. Garnreiniger werden an Textilmaschinen wie Spinn- oder Spulmaschinen zur Qualitätskontrolle am laufenden Garn eingesetzt. Ein Garnreiniger beinhaltet mindestens eine Messzelle mit einem Garnsensor zur Erfassung von Garnparametern und eine Auswerteeinheit zur Beurteilung, ob die detektierten Eigenschaften bestimmten Qualitätskriterien genügen oder nicht. Ein möglicher Aufbau eines Garnreinigers ist in der US-6,422,072 B1 offenbart. Fakultativ kann der Garnreiniger auch eine Schneidvorrichtung zur Entfernung qualitativ ungenügender Garnabschnitte enthalten.

Die detektierten Garnparameter sind typischerweise Masse pro Längeneinheit, Dicke, Materialzusammensetzung, Vorhandensein von festen Fremdstoffen, Farbe und/oder Haarigkeit. Zur Detektion der Garnparameter sind verschiedene Sensorprinzipien bekannt; die Verwendung eines bestimmten Sensorprinzips hängt unter anderem auch davon ab, welche Eigenschaft optimal detektiert werden soll. Die am häufigsten verwendeten Sensorprinzipien sind das kapazitive und das optische. Beim kapazitiven Garnreiniger durchläuft das Garn einen Messkondensator. Der Messkondensator misst dielektrische Eigenschaften des Garns, woraus sich bspw. die Garnmasse im Messkondensator oder die Gammaterialzusammensetzung bestimmen lässt. Ein Beispiel für einen kapazitiven Garnreiniger ist in der EP-0'924'513 A1 angegeben. Beim optischen Garnreiniger wird das Garn von einer Lichtquelle beleuchtet, und mit dem Garn wechselwirkendes Licht wird von einem Lichtdetektor detektiert. Daraus lässt sich bspw. die Garndicke oder das Vorhandensein von Fremdstoffen bestimmen. Die WO-93/13407 A1 gibt ein Beispiel für einen optischen Garnreiniger an.

Aus der EP-1'808'690 A1 und der EP-1'359'108 A1 sind Vorrichtungen zur Untersuchung von Garn bekannt, die mehrere Garnsensoren beinhalten. Die Garnsensoren liegen jeweils in einer gemeinsamen Ebene, die senkrecht zur Garnlängsrichtung steht. Somit tasten sie das Garn an derselben Längsposition aus verschiedenen Richtungen ab.

Es können auch mehrere Messzellen in ein und derselben Vorrichtung zum Einsatz kommen. Die US-3,009,101 A offenbart eine gattungsgemässe Vorrichtung mit zwei kapazitiven Messzellen, die auf einer gemeinsamen, isolierten Basisplatte befestigt sind, wobei das Garn parallel zur Basisplatte verläuft. Beispiele für Vorrichtungen mit je einem optischen Fremdstoffsensor und einem kapazitiven Massensensor geben die US-6,422,072 B1, die US-5,054,317 A und die WO-01/92875 A1 an. Die EP-0'761'853 A1 offenbart einen aus mehreren nach unterschiedlichen Messprinzipien arbeitenden Teilen zusammengesetzten Messkopf. Die WO-00/07921 A1 beschreibt eine Vorrichtung mit einem U-förmigen Messspalt, an dessen Seitenwänden zwei Messzellen in Garnlaufrichtung hintereinander befestigt sind, die jeweils kapazitiv oder optisch sein können.

Gemäss der US-6,422,072 B1 ist im Bereich eines Messspaltes für das Prüfgut eine Messzelle auf einer Leiterplatte angeordnet, die einen Parameter gemäss einem ersten Messprinzip erfasst. Der Messspalt weist eine zweite Messzelle auf, die mit einem anderen Messprinzip arbeitet und die auf einer zweiten Leiterplatte angeordnet ist. Beide Leiterplatten sind in einem gemeinsamen Gehäuse gelagert. Ein Nachteil dieser bekannten Vorrichtung ist darin zu sehen, dass die beiden Messzellen zusammen einen gemeinsamen Messspalt bilden sollen und deshalb genau aufeinander ausgerichtet sein müssen. Es muss darauf geachtet werden, dass die beiden Messzellen nicht gegeneinander versetzt sind oder nicht in einem Winkel zueinander stehen. Andernfalls würde die Vergleichbarkeit der Messungen in beiden Messzellen beeinträchtigt. Um diese Nachteile zu vermeiden, sind geeignete konstruktive Massnahmen zu treffen, d. h. die Messzellen müssen auf den beiden Leiterplatten genau positioniert und ausgerichtet sein und die Leiterplatten müssen ihrerseits im Gehäuse zueinander ebenfalls genau positioniert und ausgerichtet sein. Diese Massnahmen machen die Vorrichtung aufwändig in der Herstellung und teuer. Ausserdem gelingen sie nicht immer, so dass die Gefahr ungenauer Messungen besteht.

### DARSTELLUNG DER ERFINDUNG

Es ist eine Aufgabe der vorliegenden Erfindung, eine konstruktiv einfache Vorrichtung dieser Art zu schaffen, die für die Erfassung von Parametern an Prüfgut mit mehreren Messzellen geeignet ist, genau misst und kostengünstig herstellbar ist.

Diese und andere Aufgaben werden durch die erfindungsgemässe Vorrichtung, wie sie im ersten Patentanspruch definiert ist, gelöst. Vorteilhafte Ausführungsformen sind in den abhängigen Patentansprüchen angegeben.

Die Erfindung beruht auf der Idee, mindestens zwei Messzellen auf einem gemeinsamen ebenen Träger anzuordnen. Unter dem Begriff "Messzelle" wird in dieser Schrift eine eigenständige, in sich geschlossene Baugruppe verstanden, die mindestens einen zur Abtastung des Prüfgutes ausgebildeten Sensor enthält. Dabei ist zu beachten, dass eine Messzelle gemäss dieser Definition durchaus mehrere Elemente eines einzigen Sensors, wie z. B: zwei Elektroden eines kapazitiven Sensors oder Lichtsender und Lichtempfänger eines optischen Sensors, oder sogar mehrere Sensoren enthalten kann.

Die erfindungsgemässe Vorrichtung dient zur Erfassung von Parametern an einem fadenförmigen Prüfgut, das in seiner Längsrichtung durch einen Messspalt bewegt wird. Der Messspalt weist eine erste Messzelle und eine zweite Messzelle zur Erfassung von Parametern auf. Die erste Messzelle und die zweite Messzelle sind in Längsrichtung des Prüfgutes hintereinander angeordnet. Die erste Messzelle und die zweite Messzelle sind auf einem gemeinsamen ebenen Träger, dessen Ebene senkrecht zur Längsrichtung des Prüfgutes steht, angeordnet.

Der ebene Träger ist vorzugsweise eine Leiterplatte (printed circuit board, PCB), d. h. ein Träger für elektronische Bauteile mit daran haftenden Leiterbahnen. Die erste Messzelle und die zweite Messzelle können zur Erfassung derselben oder verschiedener Parameter ausgebildet sein. Sie können auf denselben oder auf unterschiedlichen Messprinzipien beruhen. Besonders vorteilhaft ist es, wenn eine der beiden Messzellen auf einem optischen Messprinzip und die andere der beiden Messzellen auf einem kapazitiven Messprinzip beruht. Die beiden Messzellen können bezüglich des Trägers in zwei verschiedenen Halbräumen oder im selben Halbraum angeordnet sein. Eine der Messzellen kann sich über beide Flächen des Trägers erstrecken.

In einer bevorzugten Ausführungsform ist die erste Messzelle auf dem Träger befestigt, während die zweite Messzelle auf der ersten Messzelle befestigt ist. Durch eine solche "sequenzielle" Anordnung der Messzellen wird sichergestellt, dass die beiden Messzellen genau aufeinander ausgerichtet sind, ohne dass mechanische Spannungen entstehen.

Bei der erfindungsgemässen Vorrichtung können mehr als zwei Messzellen auf dem gemeinsamen Träger angeordnet sein. Die Vorrichtung kann auch mehr Träger als den gemeinsamen Träger beinhalten.

Die Erfindung umfasst auch einen elektronischen Garnreiniger mit einer Vorrichtung zur Erfassung von Parametern an einem Garn und einer Auswerteeinheit zur Beurteilung, ob die detektierten Eigenschaften bestimmten Qualitätskriterien genügen oder nicht. Die Vorrichtung zur Erfassung von Parametern ist eine erfindungsgemässe Vorrichtung, wie sie in dieser Schrift beschrieben ist. In einer bevorzugten Ausführungsform beinhaltet der erfindungsgemässe Garnreiniger einen einzigen Träger mit mindestens zwei Messzellen. Der Träger ist vorzugsweise eine Leiterplatte, auf welcher sich die Auswerteeinheit befinden kann.

Die durch die Erfindung erreichten Vorteile sind insbesondere darin zu sehen, dass, um einen oder mehrere verschiedene Parameter an einem Prüfgut zu erfassen, die Messfelder der Messzellen mit einfachen Mitteln genau zueinander ausgerichtet werden, ohne dass mechanische Spannungen zwischen den Messzellen oder diesen und dem Träger entstehen können. Damit kann auch die Dicke der Leiterplatte in einem gewissen Bereiche variieren, ohne dass dies die Genauigkeit der Montage und schliesslich der Messungen beeinträchtigt. Dank der Verwendung eines einzigen Trägers für mehrere Messzellen ist die erfindungsgemässe Vorrichtung einfach und kostengünstig in der Herstellung. Wird eine optische Messzelle direkt auf die Leiterplatte aufgeklebt, so ist sie auch gegen das Eindringen von Schmutz in das Messfeld geschützt.

### AUFZÄHLUNG DER ZEICHNUNGEN

Nachfolgend wird die Erfindung anhand der Zeichnungen detailliert erläutert.
- Figur 1: zeigt schematisch eine erste Ausführungsform der erfindungsgemässen Vorrichtung in einem Querschnitt.
- Figuren 2-4: zeigen schematisch je eine weitere Ausführungsform der erfindungsgemässen Vorrichtung in Seitenansichten.
- Figur 5: zeigt einen Teil der erfindungsgemässen Vorrichtung (a) in einer Seitenansicht und (b) in einer Ansicht von unten.

### AUSFÜHRUNG DER ERFINDUNG

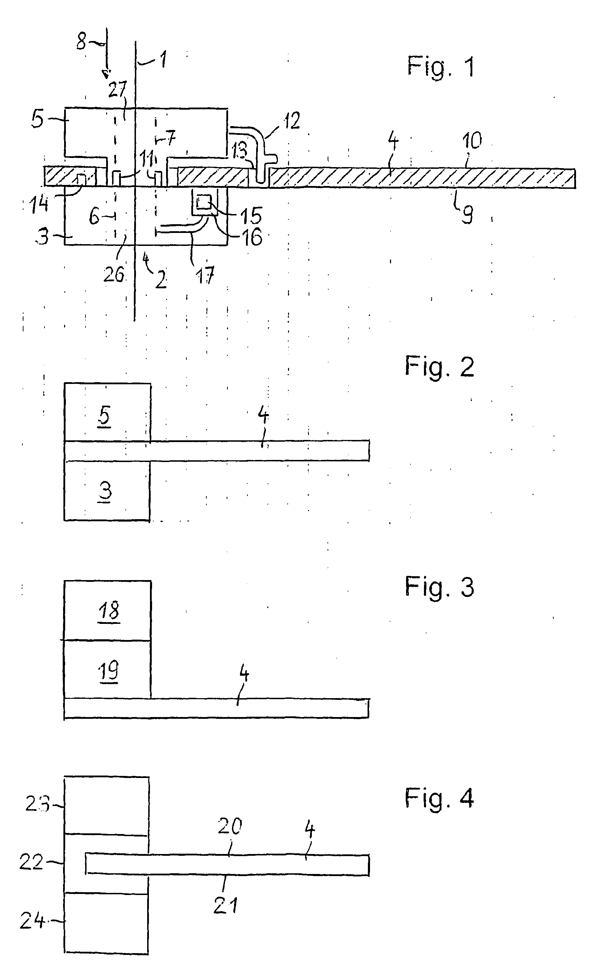

Figur 1 zeigt eine erste Ausführungsform der erfindungsgemässen Vorrichtung im Querschnitt. Die Vorrichtung weist in bekannter Weise einen Messspalt 2 für ein in der Richtung eines Pfeiles 8 längsbewegtes fadenförmiges Prüfgut 1, beispielsweise ein Garn, auf. Der Messspalt 2 ist durch unterbrochene Linien 6 und 7 angedeutet und begrenzt ein erstes Messfeld 26, bspw. ein optisches Messfeld, und ein zweites Messfeld 27, bspw. ein kapazitives Messfeld.

Eine einzige, ebene Leiterplatte 4 trägt die wesentlichen Elemente der Vorrichtung. Die Ebene der Leiterplatte 4 steht senkrecht zur Längsrichtung des Prüfgutes 1. Die Leiterplatte 4 kann eine Dicke von z. B. 1.6 mm aufweisen und beidseitig bestückt sein. Sie kann nach irgendeiner der an sich bekannten Technologien hergestellt sein.

Eine erste Messzelle 3, die das erste Messfeld 26 beherbergt, ist direkt auf einer ersten Fläche 9 der Leiterplatte 4 befestigt oder angeordnet. Die erste Messzelle 3 ist beispielsweise auf die Leiterplatte 4 aufgeklebt oder auf andere Art befestigt, wobei mindestens ein Stift 14 zur Positionierung der ersten Messzelle 3 auf der Leiterplatte 4 vorgesehen ist, der in eine geeignete Bohrung in der Leiterplatte 4 formschlüssig eingreift. Im Bereich der ersten Messzelle 3 kann, wenn es sich beim ersten Messfeld 26 um ein optisches Messfeld handelt, ein optisches Element 15, z. B. eine Photodiode oder eine Leuchtdiode (LED), auf der Leiterplatte 4 angebracht sein. Solche optischen und andere Elemente sind vorzugsweise als oberflächenmontierbare Bauteile (surface-mounted device, SMD) auf der Leiterplatte 4 montiert, d. h. sie werden mittels lötfähiger Anschlussflächen direkt auf die Leiterplatte gelötet. An das optische Element 15 kann ein Wellenleiter 17 angeschlossen sein, über den Licht vom optischen Messfeld 26 zum optischen Element 15 und/oder umgekehrt geführt wird. Ein Zwischenraum 16 zwischen dem optischen Element 15 und dem Wellenleiter 17 ist vorzugsweise mit einem optischen Klebstoff, der für die verwendeten Lichtwellenlängen transparent ist, ausgefüllt, so dass das optische Element 15 vor Verschmutzung geschützt ist.

Eine zweite Messzelle 5, die das zweite Messfeld 27 beherbergt, ist auf der zweiten Fläche 10 der Leiterplatte 4 angeordnet. Die zweite Messzelle 5 ist auf der ersten Messzelle 3 durch Positionier- und Befestigungsstifte 11 formschlüssig zentriert bzw. positioniert und befestigt. Allerdings kann die Befestigung alternativ oder zusätzlich durch Kleben und/oder andere Mittel erfolgen, so dass die Befestigungsstifte 11 nur für die Positionierung sorgen können. Ein Kontaktelement 12 der zweiten Messzelle 5 mündet in eine Bohrung 13 in der Leiterplatte 4, um einen elektrischen Kontakt zwischen der Messzelle 5 und Leiterbahnen auf der Leiterplatte 4 herzustellen. Die Bohrung 13 ist im Vergleich zum Kontaktelement 12 grosszügig bemessen, so dass beim Einführen des Kontaktelementes 12 in die Bohrung 13 keine mechanischen Spannungen entstehen. Die Bohrung ist vorzugsweise mit Lötmaterial (Lot) ausgefüllt. Diese Durchloch-Technologie (through-hole technology, THT) ist für die Kontaktierung von Komponenten auf Leiterplatten an sich bekannt und deshalb hier nicht näher erläutert.

Ein Vorteil der Ausführungsform von Figur 1 besteht darin, dass die erste Messzelle 3 direkt auf der Leiterplatte 4 befestigt ist, während die zweite Messzelle 5 nicht direkt auf der Leiterplatte, sondern auf der ersten Messzelle 3 befestigt ist. Dadurch können die beiden Messfelder 26, 27 genau aufeinander ausgerichtet werden, ohne dass mechanische Spannungen in der Vorrichtung entstehen. Dank dieser Anordnung der Messzellen 3, 5 können auch relativ grosse Abweichungen von bspw. ±10 % ≈ ±0.16 mm toleriert werden, ohne dass dadurch die Montagegenauigkeit leidet. Die grösseren Toleranzen erlauben es auch, preisgünstigere Leiterplatten für die erfindungsgemässe Vorrichtung zu verwenden, was die Vorrichtung preisgünstig macht.

Figur 2 zeigt schematisch eine zweite Ausführungsform der erfindungsgemässen Vorrichtung. Hier sind die beiden Messzellen 3 und 5, wie schon aus der Fig. 1 bekannt, beidseitig der Leiterplatte 4 angeordnet. Der wesentliche Unterschied zur ersten Ausführungsform von Figur 1 besteht darin, dass jede der beiden Messzellen 3, 5 direkt auf der Leiterplatte 4 befestigt ist.

Eine dritte Ausführungsform der erfindungsgemässen Vorrichtung ist schematisch in Figur 3 dargestellt. Darin sind zwei Messzellen 18 und 19, deren Funktion und Aufbau analog zu den Messzellen 3, 5 der Figuren 1 und 2 sein können, auf derselben Seite der Leiterplatte 4 angeordnet. In Analogie zur Ausführungsform von Figur 1 ist nur eine erste Messzelle 19 direkt auf der Leiterplatte 4 befestigt, während eine zweite Messzelle 18 direkt auf der ersten Messzelle 19, aber nur indirekt auf der Leiterplatte 4 befestigt ist. Vorteile einer solchen Anordnung von Messzellen 18, 19 wurden anlässlich der Figur 1 erwähnt.

Figur 4 zeigt eine vierte Ausführungsform der erfindungsgemässen Vorrichtung. Diese beinhaltet drei Messzellen 22-24. Eine erste Messzelle 22 erstreckt sich über beide Flächen 20, 21 der Leiterplatte 4 und umfasst so einen Teil der Leiterplatte 4 in ihrem Randbereich. Zwei weitere Messzellen 23, 24 sind direkt an der ersten Messzelle 22 befestigt.

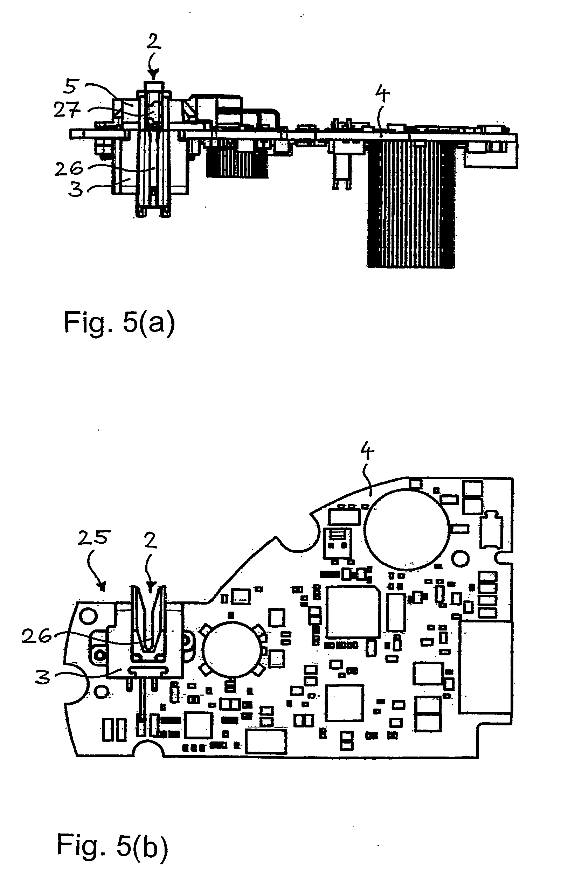

Figuren 5(a) und (b) zeigen eine Ausführungsform der erfindungsgemässen Vorrichtung in einer Seitenansicht bzw. in einer Ansicht von unten. Man erkennt den Messspalt 2 und die beiden Messzellen 3, 5, die in einem Randbereich 25 der Leiterplatte 4 angeordnet sind. Somit lässt sich das Garn in Bezug auf die Leiterplatte 4 seitlich in den Messspalt 2 einlegen. Wie aus diesen Figuren ersichtlich, trägt die Leiterplatte 4 nebst den Messzellen 3, 5 noch viele weitere Komponenten, insbesondere elektronische Schaltungskomponenten, integrierte Schaltkreise, Leiterbahnen, Anschlüsse etc. Diese weiteren Komponenten dienen vorzugsweise der Steuerung und Stromversorgung der Messzellen sowie der Verarbeitung und Auswertung der Ausgangssignale der Messzellen. Vorzugsweise befindet sich somit eine Auswerteeinheit zur Beurteilung, ob die von den Messzellen 3, 5 detektierten Garneigenschaften bestimmten Qualitätskriterien genügen oder nicht, auf der Leiterplatte 4. Die weiteren Komponenten auf der Leiterplatte 4 können aber weitere Funktionen haben, wie z. B. die Steuerung und Stromversorgung einer (nicht eingezeichneten) Garnschneidvorrichtung. Diese Vorrichtung wird vorzugsweise in einem elektronischern Garnreiniger eingesetzt. Ein besonders einfacher Aufbau des Garnreinigers ergibt sich, wenn er eine einzige Leiterplatte 4 mit mindestens zwei Messzellen 3, 5 beinhaltet.

Nachfolgend wird die Wirkungsweise der erfindungsgemässen Vorrichtung beschrieben, sofern sie sich nicht schon aus der obigen Beschreibung ergibt. Um beispielsweise in einem textilen Garn 1 in an sich bekannter und deshalb hier nicht näher dargestellter Weise einerseits Abweichungen des Durchmessers oder der Masse von einem Mittelwert und andererseits im selben Durchgang Fremdstoffe wie z. B. Fremdfasern zu bestimmen, die im Garn 1 eingelagert sein können, weist die Vorrichtung mindestens zwei Messzellen 3 und 5 auf, die an einen einzigen Messspalt 2 angrenzen und vorzugsweise nach unterschiedlichen Messprinzipien arbeiten. Dazu kann eine erste Messzelle 3 z. B. optisch arbeiten, um Farbunterschiede im Garn 1 festzustellen, die durch Fremdstoffe erzeugt sind. Eine zweite Messzelle 5 kann beispielsweise kapazitiv arbeiten und Änderungen der Masse des Garns 1 feststellen. Die Messzellen 3, 5 an sich sowie Schaltungen und Verfahren zur Auswertung ihrer Signale sind aus dem Stand der Technik hinreichend bekannt und brauchen hier nicht weiter erklärt zu werden.

### BEZUGSZEICHENLISTE

- 1: Prüfgut
- 2: Messspalt
- 3: erste Messzelle
- 4: Leiterplatte
- 5: zweite Messzelle
- 6, 7: Begrenzungen des Messspaltes
- 8: Bewegungsrichtung des Prüfgutes
- 9, 10: Flächen der Leiterplatte
- 11: Positionier- und Befestigungsstifte
- 12: Kontaktelement
- 13: Bohrung
- 14: Positionierstift
- 15: optisches Element
- 16: Zwischenraum
- 17: Wellenleiter
- 18: zweite Messzelle
- 19: erste Messzelle
- 20, 21: Flächen der Leiterplatte
- 22: erste Messzelle
- 23, 24: weitere Messzellen
- 25: Randbereich der Leiterplatte
- 26: erstes Messfeld
- 27: zweites Messfeld

## Patentansprüche

1. Vorrichtung zur Erfassung von Parametern an einem fadenförmigen Prüfgut (1), das in seiner Längsrichtung (8) durch einen Messspalt (2) bewegt wird, wobei der Messspalt eine erste Messzelle (3,19,22) und eine zweite Messzelle (5,18,23,24) zur Erfassung von Parametern aufweist und
die erste Messzelle (3,19,22) und die zweite Messzelle (5,18,23,24) in Längsrichtung des Prüfgutes (1) hintereinander angeordnet sind,
**dadurch gekennzeichnet,**
**dass** die erste Messzelle (3,19,22) und die zweite Messzelle (5,18,23,24) auf einem gemeinsamen ebenen Träger (4), dessen Ebene senkrecht zur Längsrichtung (8) des Prüfgutes (1) steht, angeordnet sind.

2. Vorrichtung nach Anspruch 1, wobei der ebene Träger (4) eine Leiterplatte ist.

3. Vorrichtung nach Anspruch 2, wobei ein optisches Element (15), z. B. eine Photodiode oder eine Leuchtdiode, im Bereich einer (3) der beiden Messzellen (3, 5), die ein optisches Messfeld (26) beherbergt, auf der Leiterplatte (4) angebracht ist, an welches optische Element (15) ein Wellenleiter (17) angeschlossen ist, und wobei ein Zwischenraum (16) zwischen dem optischen Element (15) und dem Wellenleiter (17) mit einem optischen Klebstoff, der für die verwendeten Lichtwellenlängen transparent ist, ausgefüllt ist.

4. Vorrichtung nach einem der vorangehenden Ansprüche, wobei die erste Messzelle (3) und die zweite Messzelle (5) zur Erfassung verschiedener Parameter ausgebildet sind.

5. Vorrichtung nach einem der vorangehenden Ansprüche, wobei die erste Messzelle (3) und die zweite Messzelle (5) auf unterschiedlichen Messprinzipien beruhen.

6. Vorrichtung nach Anspruch 5, wobei eine (3) der der beiden Messzellen (3, 5) auf einem optischen Messprinzip und die andere (5) der beiden Messzellen (3, 5) auf einem kapazitiven Messprinzip beruht.

7. Vorrichtung nach einem der vorangehenden Ansprüche, wobei die erste Messzelle (3) und die zweite Messzelle (5) bezüglich des Trägers (4) in zwei verschiedenen Halbräumen angeordnet sind.

8. Vorrichtung nach einem der Ansprüche 1-6, wobei die erste Messzelle (19) und die zweite Messzelle (18) bezüglich des Trägers (4) im selben Halbraum angeordnet sind.

9. Vorrichtung nach einem der vorangehenden Ansprüche, wobei eine (22) der Messzellen (22-24) sich über beide Flächen (20, 21) des Trägers (4) erstreckt.

10. Vorrichtung nach einem der vorangehenden Ansprüche, wobei eine (3) der Messzellen (3, 5) formschlüssig auf dem Träger (4) positioniert ist.

11. Vorrichtung nach einem der vorangehenden Ansprüche, wobei die erste Messzelle (19) auf dem Träger (4) befestigt ist, während die zweite Messzelle (18) auf der ersten Messzelle (19) befestigt ist.

12. Vorrichtung nach einem der vorangehenden Ansprüche, wobei mehr als zwei Messzellen (22-24) auf dem gemeinsamen Träger (4) angeordnet sind.

13. Elektronischer Garnreiniger mit
einer Vorrichtung zur Erfassung von Parametern an einem Garn (1) und
einer Auswerteeinheit zur Beurteilung, ob die detektierten Eigenschaften bestimmten Qualitätskriterien genügen oder nicht,
**dadurch gekennzeichnet,**
**dass** die Vorrichtung zur Erfassung von Parametern eine Vorrichtung nach einem der vorangehenden Ansprüche ist.

14. Elektronischer Garnreiniger nach Anspruch 13, wobei die Vorrichtung einen einzigen ebenen Träger (4) mit mindestens zwei Messzellen (3, 5) beinhaltet.

15. Elektronischer Garnreiniger nach Anspruch 13 oder 14 und nach Anspruch 2, wobei sich die Auswerteeinheit auf der Leiterplatte (4) befindet.

## Claims

1. A device for detecting parameters on a thread-like product to be tested (1) which is moved in its longitudinal direction (8) through a measurement gap (2), wherein
the measurement gap comprises a first measurement cell (3, 19, 22) and a second measurement cell (5, 18, 23, 24) for detecting parameters and
the first measurement cell (3, 19, 22) and the second measurement cell (5, 18, 23, 24) are arranged one after another in the longitudinal direction of the product to be tested (1),
**characterised in that**
the first measurement cell (3, 19, 22) and the second measurement cell (5, 18, 23, 24) are arranged on a common, plane carrier (4), whose plane is perpendicular to the longitudinal direction (8) of the product to be tested (1).

2. The device according to claim 1, wherein the plane carrier (4) is a circuit board.

3. The device according to claim 2, wherein an optical element (15), e.g. a photodiode or a light emitting diode, is attached on the circuit board (4) in the region of one (3) of the two measurement cells (3, 5) which accommodates a first measurement field (26), to which optical element (15) a waveguide (17) is connected, an intermediate space (16) between the optical element (15) and the waveguide (17) being filled out with an optical adhesive which is transparent to the applied light wavelengths.

4. The device according to one of the preceding claims, wherein the first measurement cell (3) and the second measurement cell (5) are designed for detecting different parameters.

5. The device according to one of the preceding claims, wherein the first measurement cell (3) and the second measurement cell (5) are based on different measurement principles.

6. The device according to claim 5, wherein one (3) of the two measurement cells (3, 5) is based on the optical measurement principle and the other (5) of the two measurement cells (3, 5) is based on a capacitive measurement principle.

7. The device according to one of the preceding claims, wherein the first measurement cell (3) and the second measurement cell (5) are arranged in two different semi-spaces with respect to the carrier (4).

8. The device according to one of the claims 1-6, wherein the first measurement cell (18) and the second measurement cell (19) are arranged in the same semi-space with respect to the carrier (4).

9. The device according to one of the preceding claims, wherein one (22) of the measurement cells (22-24) extends over both surfaces (20, 21) of the carrier (4).

10. The device according to one of the preceding claims, wherein one (3) of the measurement cells (3, 5) is positioned on the carrier (4) with a positive fit.

11. The device according to one of the preceding claims, wherein the first measurement cell (3) is fastened on the carrier (4), whilst the second measurement cell (5) is fastened on the first measurement cell (3).

12. The device according to one of the preceding claims, wherein more than two measurement cells (22-24) are arranged on the common carrier (4).

13. An electronic yarn clearer with
a device for detecting parameters on a yarn (1) and
with an evaluation unit for assessing whether the detected characteristics meet certain quality criteria or not,
**characterised in that**
the device for detecting parameters is a device according to one of the preceding claims.

14. The electronic yarn clearer according to claim 13, wherein the device contains a single plane carrier (4) with at least two measurement cells (3, 5).

15. An electronic yarn clearer according to claim 13 or 14 and according to claim 2, wherein the evaluation unit is located on the circuit board (4).

## Revendications

1. Dispositif pour l'acquisition de paramètres sur une matière à contrôler (1) en forme de fil qui est déplacée dans son sens longitudinal (8) à travers une fente de mesure (2), dans lequel
la fente de mesure comporte une première cellule de mesure (3, 19, 22) et une deuxième cellule de mesure (5, 18, 23, 24) pour l'acquisition de paramètres, et
la première cellule de mesure (3, 19, 22) et la deuxième cellule de mesure (5, 18, 23, 24) sont disposées l'une derrière l'autre dans le sens longitudinal de la matière à contrôler (1),
**caractérisé en ce que**
la première cellule de mesure (3, 19, 22) et la deuxième cellule de mesure (5, 18, 23, 24) sont disposées sur un support plan (4) commun, dont le plan est perpendiculaire au sens longitudinal (8) de la matière à contrôler (1).

2. Dispositif selon la revendication 1, dans lequel le support plan (4) est une carte de circuits.

3. Dispositif selon la revendication 2, dans lequel un élément optique (15), par exemple une photodiode ou une diode électroluminescente, est disposé sur la carte de circuits (4) dans la zone de l'une (3) des deux cellules de mesure (3, 5) qui abrite un champ de mesure optique (26), auquel élément optique (15) se raccorde un guide d'ondes (17), et dans lequel un espace intermédiaire (16) entre l'élément optique (15) et le guide d'ondes (17) est rempli avec un adhésif optique qui est transparent aux longueurs d'ondes lumineuses utilisées.

4. Dispositif selon l'une des revendications précédentes, dans lequel la première cellule de mesure (3) et la deuxième cellule de mesure (5) sont conçues pour l'acquisition de différents paramètres.

5. Dispositif selon l'une des revendications précédentes, dans lequel la première cellule de mesure (3) et la deuxième cellule de mesure (5) reposent sur des principes de mesure différents.

6. Dispositif selon la revendication 5, dans lequel l'une (3) des deux cellules de mesure (3, 5) repose sur un principe de mesure optique et l'autre (5) des deux cellules de mesure (3, 5) sur un principe de mesure capacitif.

7. Dispositif selon l'une des revendications précédentes, dans lequel la première cellule de mesure (3) et la deuxième cellule de mesure (5) sont disposées dans deux demi-espaces différents par rapport au support (4).

8. Dispositif selon l'une des revendications 1 à 6, dans lequel la première cellule de mesure (19) et la deuxième cellule de mesure (18) sont disposées dans le même demi-espace par rapport au support (4).

9. Dispositif selon l'une des revendications précédentes, dans lequel l'une (22) des cellules de mesure (22-24) s'étend sur les deux surfaces (20, 21) du support (4).

10. Dispositif selon l'une des revendications précédentes, dans lequel l'une (3) des cellules de mesure (3, 5) est positionnée en correspondance de forme sur le support (4).

11. Dispositif selon l'une des revendications précédentes, dans lequel la première cellule de mesure (19) est fixée sur le support (4) tandis que la deuxième cellule de mesure (18) est fixée sur la première cellule de mesure (19).

12. Dispositif selon l'une des revendications précédentes, dans lequel plus de deux cellules de mesure (22-24) sont disposées sur le support (4) commun.

13. Nettoyeur de fil électronique avec
un dispositif pour l'acquisition de paramètres sur un fil (1) et
une unité d'analyse pour évaluer si les propriétés détectées satisfont ou non certains critères de qualité,
**caractérisé en ce que**
le dispositif pour l'acquisition des paramètres est un dispositif selon l'une des revendications précédentes.

14. Nettoyeur de fil électronique selon la revendication 13, dans lequel le dispositif contient un seul support plan (4) avec au moins deux cellules de mesure (3, 5).

15. Nettoyeur de fil électronique selon la revendication 13 ou 14 et selon la revendication 2, dans lequel l'unité d'analyse se trouve sur la carte de circuits (4).
